# EUROPEAN PATENT APPLICATION

(11) **EP 2 578 189 A1**
(43) Date of publication of application: **10.04.2013**
(21) Application number: 11184408.0
(22) Date of filing: 07.10.2011
(51) Int. Cl.: A61F 9/00, A61M 5/32

(54) **Apparatus for intraocular injection**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is an apparatus for intraocular injection comprising an inner sleeve (38) adapted to accommodate a syringe (32) having a needle (34), a needle cover (36) adapted to cover the needle (34), and an outer sleeve (37) telescopically coupled to the inner sleeve (38). The inner sleeve (38) includes a distal end adapted to engage the needle cover (16). Movement of the inner sleeve (38) relative to the outer sleeve (37) causes removal of the needle cover (36) from the needle (34).

## Description

The present invention relates to an apparatus for intraocular injection.

### Background

An intraocular injection device may be used to administer therapeutic substances to eyes, such as eyes of mammals having eye disorders or diseases.

A number of vision-threatening disorders or diseases of the eye need to deliver a medicament (pharmaceutical, biological, etc.) and/or implantable device to a posterior segment of the eye by intraocular delivery (more specifically intravitreal delivery). One such technique for intraocular delivery is accomplished by intraocular injection into the vitreous body.

A conventional apparatus for intraocular injection may include a pre-filled syringe of a medicament. A conventional pre-filled syringe is supplied with a needle cover in order to maintain sterility of a needle. However, the needle cover is typically frictionally held on the needle which can result in dislodging of the needle cover. If any portion of the needle becomes unsterile prior to use, the syringe must be discarded.

Therefore, there is a need for an apparatus for intraocular injection which ensures that a needle remains covered until use and facilitates removal of a needle cover.

### Summary

The exemplary embodiments of the present invention describe an apparatus for intraocular injection which ensures that a needle remains covered until use and facilitates removal of a needle cover.

In an exemplary embodiment, an apparatus for intraocular injection according to the present invention comprises an inner sleeve adapted to accommodate a syringe having a needle, a needle cover adapted to cover the needle, and an outer sleeve telescopically coupled to the inner sleeve. The inner sleeve includes a distal end adapted to engage the needle cover. Movement of the inner sleeve relative to the outer sleeve causes removal of the needle cover from the needle.

In an exemplary embodiment, the inner sleeve is moveable in longitudinal direction between a first configuration and a second configuration.

In an exemplary embodiment, the inner sleeve comprises a placement foot.

In an exemplary embodiment, a rim of the placement foot is adapted to engage the needle cover.

In an exemplary embodiment, the inner sleeve comprises a projection which prevents movement relative to the outer sleeve beyond a predetermined distance.

The person skilled in the art understands that the present invention is not restricted to the explained possibilities.

The above mentioned advantages as well as other advantages of various aspects of the present invention will become apparent to those of ordinary skill in the art by reading the following detailed description, with appropriate reference to the accompanying drawings.

### Brief Description of the Drawings

Exemplary embodiments of the present invention are described herein with reference to the schematic drawings in which:
Fig. 1 and 2 illustrate a side view and a perspective view of a cross section of an exemplary embodiment of an apparatus for intraocular injection in a first configuration;
Fig. 3 and 4 show an exemplary embodiment of an apparatus for intraocular during removal of a needle cover; and
Fig. 5 and 6 depict an exemplary embodiment of an apparatus for intraocular in a second configuration after removal of a needle cover.

### Detailed Description

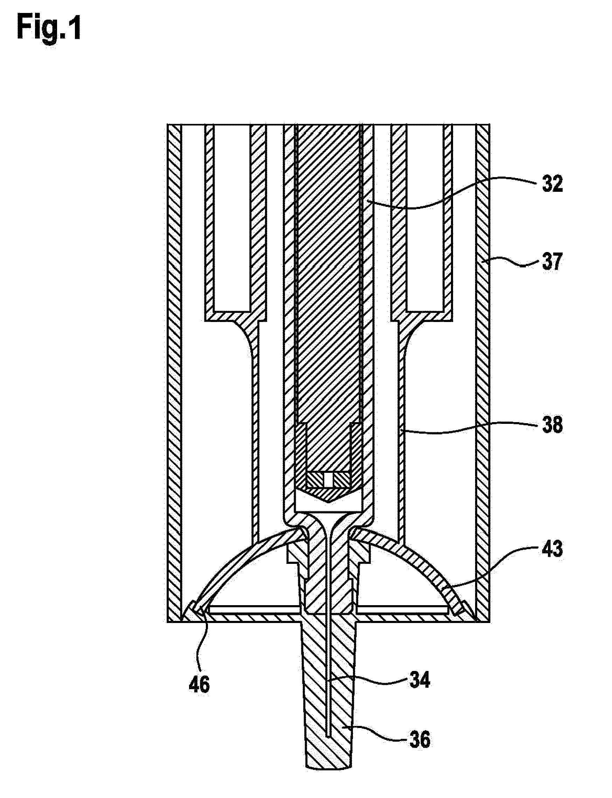

Figures 1 to 6 illustrate an exemplary embodiment of an apparatus for intraocular injection according to the present invention. In this exemplary embodiment, a body of the apparatus for intraocular injection accommodates a pre-filled syringe 32. In the exemplary embodiment, the apparatus may be an auto-injector, delivering the entire contents of the syringe 32 when the apparatus is activated. In another exemplary embodiment, the apparatus may be a reusable, fixed dose delivery device for administering only a portion of the contents of syringe 32 per injection. Those of skill in the art will understand that in another exemplary embodiment the pre-filled syringe 32 may be replaced by a medicament cartridge having a needle or having an interface for engaging a removable needle assembly. The syringe 32 comprises at its distal end a needle 34 which is protected by a needle cover 36.

In an exemplary embodiment, the apparatus comprises an outer sleeve 37 and an inner housing sleeve 38 telescopically disposed within the outer sleeve 37. In an embodiment the outer sleeve 37 and the inner sleeve 38 basically take the form of hollow cylinders. A distal end of the inner sleeve 38 may include and, for example, may be formed integrally with a placement foot 43 which is adapted for placement on a target anatomical structure, e.g., the eye. For example, as shown in the exemplary embodiments in Figures 1 to 6, the placement foot 43 takes the form of a section of a hollow sphere. Those of skill in the art will understand that the placement foot 43 may take any other suitable form. A rim 46 formed by the placement foot 43, for example the distal end of the sphere section, engages the needle cover 36, or abuts a proximal portion of the needle cover 36. In an embodiment the needle cover 36 does not only protect the needle 34 but may take, for example in its middle section, the form of a disc-shaped cap sealing the distal end of the inner sleeve 38 and/or the distal end of the placement foot 43.

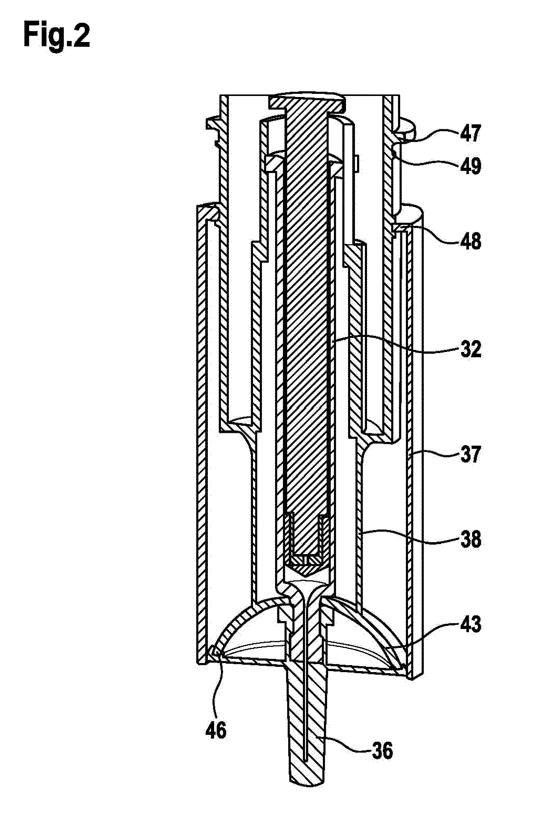

Figures 1 and 2 show an exemplary embodiment of the apparatus in a first configuration wherein the needle cover 36 covers the needle 34.

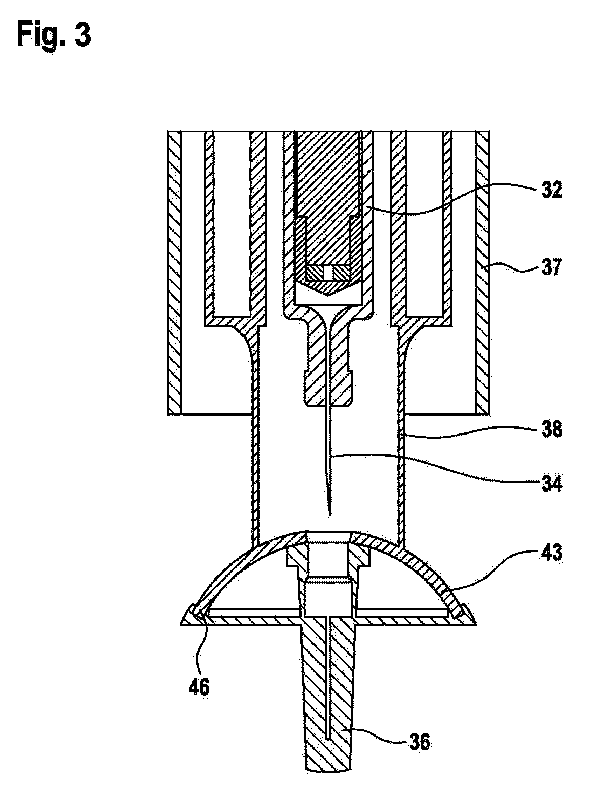

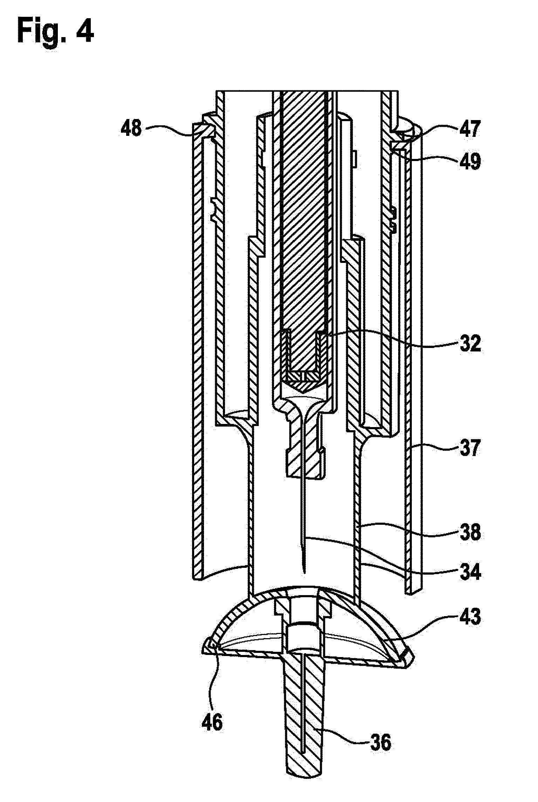

As shown in the exemplary embodiments depicted in Figures 3 and 4, when the needle cover 36 is moved distally (e.g., by pulling the needle cover 36 or advancing the inner sleeve 38), the engagement between needle cover 36 and the inner sleeve 38 causes the inner sleeve 38 to be drawn distally relative to the outer sleeve 37. A proximal end face 48 formed on a proximal end of the outer sleeve 37 may abut a projection 47 formed on a proximal portion of the inner housing sleeve 38 to prevent relative movement of the outer sleeve 37 and the inner sleeve 38 beyond a predetermined distance. The needle cover 36 can be pulled (or the inner sleeve 38 can be advanced) distally until the needle cover 36 loses its frictional connection with the needle 34 and/or the syringe 32.

A detent or latching mechanism 49, for example by a second projection, may be formed adjacent the projection 47 to engage the proximal end face 48 and to prevent relative movement of the outer sleeve 37 and the inner sleeve 38 after the needle cover 36 has been removed from the needle 34.

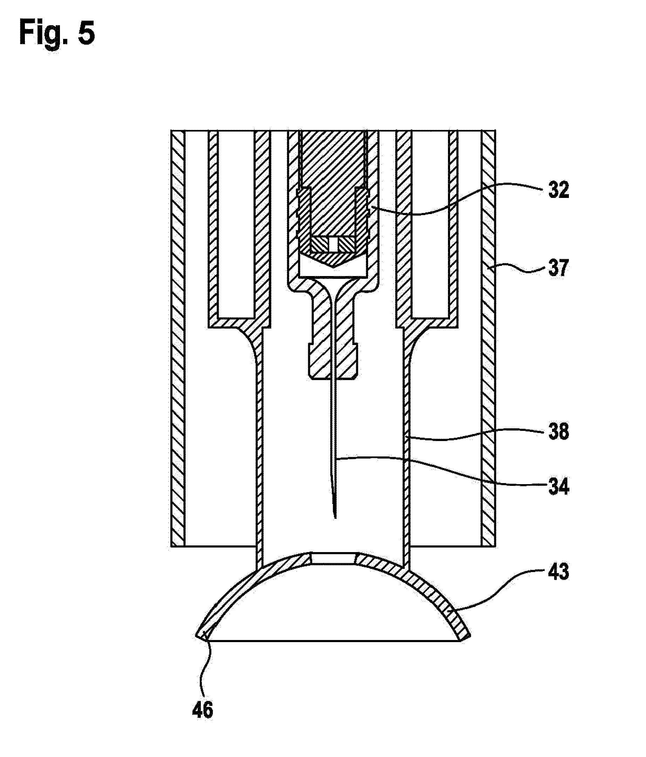

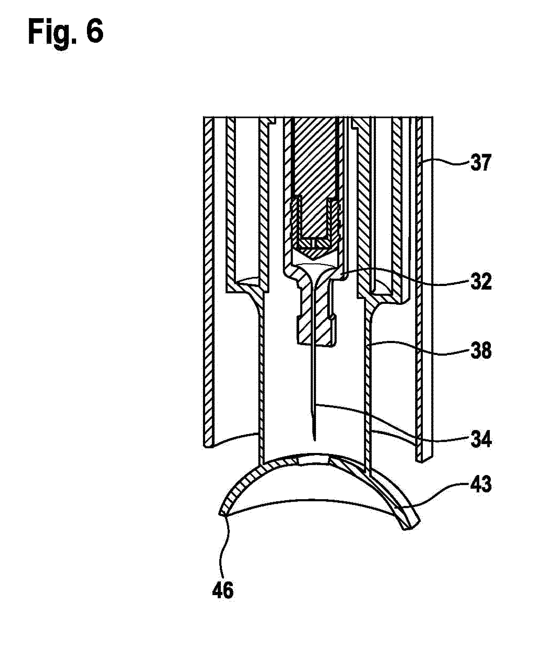

As shown in Figures 5 and 6, in the second configuration, the inner sleeve 38 may act as a needle safe feature by shielding the needle 34 even after the needle cover 36 has been removed. The movement of the inner sleeve 38 with regard to the outer sleeve 37 can be realized for example by a guide rail.

Now, using the apparatus within the second configuration the medicament may be administered to the eye.

After the needle cover 36 has been removed, the apparatus can be placed on the eye and positioned properly using the placement foot 43 to administer a medicament contained within the syringe 32. In an exemplary embodiment, when the apparatus is activated, the syringe 32 moves distally within the body so the needle 34 projects distally from the placement foot 43 and into the eye. After the injection is administered, a spring or other mechanism may be used to retract the needle 34 and/or the syringe 32 into the body such that the needle 34 is again shielded by the inner sleeve 38.

Those of skill in the art will understand that the placement foot 43 of the exemplary embodiments may be made from an at least partially transparent material such that alignment with the eye, e.g. a periphery of the cornea, may be facilitated. Further, those of skill in the art will understand that an underside of the placement foot 43, for example a surface of the foot 43 which contacts the eye may include a frictional layer or other means for gripping, without injury, the eye.

When the apparatus has been properly placed on the eye, the physician may depress a plunger or similar depressable element coupled to the body and/or the syringe 32 which advances the syringe 32 distally within the body towards the injection site. Then a medicament may be delivered to the predetermined region of the eye.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. An apparatus for intraocular injection comprising:
an inner sleeve (38) adapted to accommodate a syringe (32) having a needle (34);
a needle cover (36) adapted to cover the needle (34); and
an outer sleeve (37) telescopically coupled to the inner sleeve (38),
wherein the inner sleeve (38) includes a distal end adapted to engage the needle cover (16), and
wherein movement of the inner sleeve (38) relative to the outer sleeve (37) causes removal of the needle cover (36) from the needle (34).

2. The apparatus according to claim 1 wherein the inner sleeve (38) is moveable in longitudinal direction between a first configuration and a second configuration.

3. The apparatus according to any of the preceding claims wherein the inner sleeve (38) comprises a placement foot (43).

4. The apparatus according to any of the preceding claims wherein a rim (46) of the placement foot (43) is adapted to engage the needle cover (36).

5. The apparatus according to any of the preceding claims wherein the inner sleeve (38) comprises a projection (47) which prevents movement relative to the outer sleeve (37) beyond a predetermined distance.
